Europäisches Patentamt

European Patent Office

Office européen des brevets

(11) Publication number: **0 246 824 B1**

# EUROPEAN PATENT SPECIFICATION

(45) Date of publication of patent specification: **23.03.94**   (51) Int. Cl.⁵: **A01N 1/02**, C12N 1/04

(21) Application number: **87304330.1**

(22) Date of filing: **15.05.87**

(54) **Biological cryoprotection.**

(30) Priority: **15.05.86 GB 8611894**

(43) Date of publication of application:
**25.11.87 Bulletin  87/48**

(45) Publication of the grant of the patent:
**23.03.94 Bulletin  94/12**

(84) Designated Contracting States:
**AT BE CH DE ES FR GB GR IT LI LU NL SE**

(56) References cited:

BIOLOGICAL ABSTRACTS, vol. 65, no. 5, 1st March 1978, page 2960, abstract no. 30258, Philadelphia, PA, US; H.D.M. MOORE et al.: "Fertility of boar spermatozoa after freezing in the absence of seminal vesicular proteins"

CHEMICAL ABSTRACTS, vol. 88, no. 22, 29th May 1978, page 625, abstract no. 161594d, Columbus, Ohio, US; T. MONTEFINALE et al.: "Two-step ice nucleation"

CHEMICAL ABSTRACTS, vol. 100, no. 15, 9th April 1984, page 377, abstract no. 118544s, Columbus, Ohio, US; R.K. PANDIT et al.: "Preservation of buffalo semen at ultra-low temperature"

(73) Proprietor: **CELL SYSTEMS LTD**
**Cambridge Science Park**
**Milton Road**
**Cambridge CB4 4FY(GB)**

Proprietor: **BOURN HALL LIMITED**
**Bourn Hall Clinic**
**Bourn Hall**
**Bourn**
**Cambridge CB3 7TR(GB)**

(72) Inventor: **Morris, George John**
**36 High Street**
**Fen Ditton Cambridge CB5 8ST(GB)**
Inventor: **Ashwood-Smith, Michael John**
**Manor Cottage**
**High Street**
**Great Eversden Cambridge(GB)**

(74) Representative: **Sheard, Andrew Gregory et al**
**Kilburn & Strode**
**30, John Street**
**London WC1N 2DD (GB)**

**Description**

This invention relates to the cryoprotection of biological samples. The term "biological sample" as used in this specification includes cells (both eukaryotic and prokaryotic), organs and tissue composed of cells, viruses, all of which can be natural or genetically or otherwise modified, and biologically active molecules such as macromolecules including nucleic acids, protein, glycoproteins, lipids, lipoproteins, by way of example. The invention finds particular application in the cryoprotection of human embryos and embryos of other mammals.

The storage of cells in the frozen state (cryopreservation) is a technique commonly used for the long-term maintenance of cellular viability and genetic stability. However, a major problem encountered during cooling of samples for cryopreservation is that the medium containing the biological sample tends to supercool, whereafter ice nucleation occurs below the freezing point of the medium.

Cryopreservation media generally contain a cryoprotectant such as glycerol or dimethylsulphoxide (DMSO or $Me_2SO$). The freezing point of the medium containing the biological sample during cooling varies with the concentration of the cryoprotectant. For example, for one medium (PB1) containing 1.0, 1.5 and 2.0 M DMSO, the freezing points are approximately -2.5, -3.5 and -4.5°C respectively. When these media are cooled at the slow rates required for preservation of many biological samples, including embryos, ice nucleation rarely occurs at the freezing point of the medium. Samples may supercool to temperatures as low as -21°C. Excessive degrees of supercooling have a deleterious effect on the survival of some biological samples when they are left to continue cooling in the same vessel after ice formation. For example, the survival of eight-cell mouse embryos cooled at 0.5°C per minute falls dramatically when ice forms in the suspending medium below -7°C; and none survives supercooling to -12°C or below (see D.G. Whittingham "The Freezing of Mammalian Embryos" Elsevier (1977) page 99).

The reason for this low survival rate is believed to be as follows. At ice formation, the latent heat of fusion causes the sample temperature to rise close to that of the melting point of the medium. Simultaneously, the temperature of the bath (or other surroundings) is continuing to fall at a constant rate; therefore, the greater the degree of supercooling, the larger the temperature difference that will exist between the cooling vessel and the sample immediately after ice formation. This, in turn, will increase the cooling rate of the sample above the normal optimal rate for survival of the sample (particularly in the case of an embryo), until thermal equilibrium is reestablished with the surroundings. It has been found experimentally (D.G. Whittingham "Principles of Embryo Preservation" in "Low Temperature Preservation in Medicine and Biology", (M.J. Ashwood-Smith and J. Farrant, eds.) Pitman Medical Press, Tunbridge Wells (1980) page 71) that embryos seeded with ice at -5°C and -12°C cool at rates of less than 2°C per minute and greater than 6°C per minute, respectively, before regaining the temperature of the cooling bath or other surroundings. Embryos seeded with ice at -12°C are still viable when thawed after reaching the temperature of the cooling bath but they do not survive after continuous slow cooling to -80°C and transfer to liquid nitrogen, unless the rapid temperature drop is prevented by transfer to another cooling bath immediately after seeding.

In the particular case of embryos, survival depends upon the amount of shrinkage due to dehydration that takes place in response to the increasing concentrations of solutes as water freezes out of the suspending medium during cooling. Supercooled samples cannot dehydrate until ice forms, and subsequently the biological sample (for example the embryos) have insufficient time to dehydrate adequately before reaching the temperature where ice forms intracellularly, i.e. if they remain in the same cooling bath after ice induction.

It has been demonstrated that many cell types, not just mammalian embryos (although they require particular care), are at risk when supercooled as intracellular ice formation is invariably a consequence; this is generally lethal to the cell on thawing.

There are currently four methods in general use to avoid the problem of supercooling. Samples are presently generally cooled to temperatures of about 1 to 2°C below the freezing point of the system, and then ice nucleation is initiated by either (a) mechanical shaking, (b) thermoelectric shock, (c) thermal shock or (d) direct addition of ice crystals.

Mechanical shaking is a time consuming and cumbersome process which would be best avoided if possible. The thermoelectric shock can be delivered by supplying a current across the container (for example, an ampoule) in which the biological sample is contained: the technique uses the reverse of the Peltier thermocouple effect. Thermal shock is achieved either by contact of the outside of the container with a much colder surface, such as a cold metal bar, or the insertion of a precooled surface such as a metal wire or glass rod. Perhaps the least inelegant of the present processes is the direct addition of ice crystals. However, if many separate samples are being frozen the process is impracticable. In addition, sterility tends to be lost by the manual addition of ice crystals.

All these processes are expensive when means for automatically carrying them out are incorporated into biological freezers. In the case of the manual counterparts of the existing techniques, direct intervention and interruption of the cooling process is necessitated. There is therefore a need for an improved method of avoiding the serious consequences of supercooling and subsequent nucleation.

H.D.M. Moore and K.G. Hibbitt, *J. Reprod. Fertil.* **80(2)** 349-352 (1977) relates to the freezing of spermatazoa using an aqueous medium containing colloidal cholesterol as a cryoprotectant.

According to a first aspect of the present invention, there is provided a method of cryopreserving a biological sample, the method comprising cooling the sample in an aqueous medium in contact with crystalline cholesterol which causes water in the aqueous medium to be nucleated at the freezing point of the medium or sufficiently close to the freezing point not to cause significant damage to the sample.

Although some damage is bound to occur in freezing, the prevention of supercooling will minimise the damage associated with freezing and subsequent thawing.

The crystalline cholesterol may be sufficiently non-toxic to the biological sample.

The sample will generally be cooled in the presence of a cryoprotectant such as DMSO or glycerol or mixtures of them either alone or in conjunction with other cryoprotectants.

Ice nucleators have been known for several years, either as physico-chemical curiosities or in meteorological applications as initiators of precipitation. For cloud seeding meteorological purposes, inorganic substances such as silver iodide have probably been studied the most, although Head discussed the use of sterols as ice nucleators in cloud seeding operations some 25 years ago (Head, Nature 9th September 1961 1058 and J. Phys. Chem. Solids 23 (1962) 1371). Fukuta and Mason studied the epitaxial growth of ice on organic crystals (J. Phys. Chem. Solids 24 (1963) 715), but did not suggest any useful application for their observations. It should be pointed out that nucleation of ice by organic compounds represents a very small proportion of the work carried out on ice nucleation for meteorological purposes. For example, the textbook "Ice Physics" by Hobbs (Clarendon Press, Oxford, 1974), which is almost 1000 pages long has only a page or two on organic ice nuclei. In any event, none of the above literature even remotely suggests that ice nucleating substances, particularly organic ones, could be used to overcome a difficult problem in the cryo-preservation of biological samples.

Preferably the crystalline cholesterol for use in the present invention will cause ice to nucleate at temperatures above 7°C below the freezing point of the aqueous medium containing the biological sample, although if it causes ice to nucleate at temperatures above 10°C below the freezing point this may generally be satisfactory. Nucleation at temperatures above 5°C below the freezing point or even 3°C or 2.5°C below the freezing point will be especially preferred.

The cholesterol may be brought into contact with the medium (or the sample itself) while in the form of discrete crystals, although it is preferred that it is supplied on an inert carrier. One particularly preferred embodiment is when the cholesterol is at least partially coated on an inside surface of a suitable cryopreservation vessel such as an ampoule, a cryo- preservation tube or a cryopreservation straw. A suitable medium for the whole or at least the inside surface of the cryopreservation vessel is glass or a biologically appropriate polymer, for example, plastics material such as polypropylene or polyvinyl chloride.

Alternatively or in addition, the cholesterol may be at least partially coated onto a particulate carrier, which may be glass or biologically appropriate polymer beads. The beads may be made of modified dextran such as CYTODEX, available from Pharmacia. The coated beads can be added to the cryopreservation medium. The coating density of the cholesterol on the substrate will generally be at least 0.0007 mg/mm$^2$, with the upper limit being determined by practical convenience. At least 0.001 mg/mm$^2$ is preferred, for example less than 0.1 mg/mm$^2$. About 0.0035 mg/mm$^2$ appears to be optimal.

In general, however, the most preferred method of supplying the crystalline cholesterol is to coat it at least partially onto a substrate, and so, according to a second aspect of the present invention, there is provided a substrate at least partially coated with crystalline cholesterol which causes water in the aqueous medium to be nucleated at the freezing point of the medium or sufficiently close to the freezing point not to cause significant damage to the sample. Preferred substrates are at least the inner surfaces of ampoules, tubes, straws, and glass or polymer beads. Other preferred features of the second aspect of the invention are as for the first aspect, mutatis mutandis.

As the cholesterol will frequently be used in a cryopreservation medium in practice, the present invention also provides, in a third aspect, an aqueous cryopreservation medium for a biological sample, the medium comprising a source of crystalline cholesterol which causes water in the aqueous medium to be nucleated at the freezing point of the medium or sufficiently close to the freezing point not to cause significant damage to the sample. Other preferred features of the third aspect of the invention are as for the first and second aspects, mutatis mutandis.

3

To illustrate the present invention, the following examples are given.

Example Series I - Ice Nucleation by Organic Chemicals.

All chemicals were obtained from Sigma Chemicals. For the initial screen of ice nucleating activity, all chemicals were made up at 5 mg/ml, those marked with an asterisk being dissolved in ethanol and all others in distilled water. To plastic ampoules (Eppendorf) was added 0.1 ml solution. The solvent was removed by placing the ampoules in a water bath at 70°C for ethanol (or 90°C for distilled water). The ampoules were then thoroughly dried in an oven at 45°C. Distilled water (0.5 ml) was added to each ampoule, which was then placed in a low temperature bath (Fryka KB 300) maintained at 0°C. After five minutes to allow thermal equilibration, the bath was cooled at a rate of 0.75°C per min.; a digital thermometer (Comark 5000) was used to measure the temperature of the bath. At various bath temperatures (-2.5°, -5°, -7.5° and -10°C) ampoules were carefully removed and examined optically for the presence of ice crystals. For each additive examined, there were five replicates.

The results are shown in Table 1, in which the data are presented as number of tubes nucleated/number remaining fluid at any temperature. The crystalline cholesterol used in Example 1.1 is in accordance with the invention; the other organic solids are provided by way of comparison.

TABLE 1

| Example | Solid | Bath Temperature (°C) | | | |
|---------|-------|------|------|------|------|
| | | -2.5 | -5 | -7.5 | -10 |
| 1.1 | Cholesterol* | 0/5 | 5/0 | | |
| 1.2 | Pregnenolone* | 0/5 | 0/5 | 4/1 | 5/0 |
| 1.3 | Methyl Androsterone* | 0/5 | 0/5 | 2/3 | 5/0 |
| 1.4 | 17 alpha-Oestradiol* | 0/5 | 0/5 | 0/5 | 2/3 |
| 1.5 | Androsterone* | 0/5 | 1/4 | 5/0 | |
| 1.6 | Testosterone* | 0/5 | 0/5 | 1/4 | 5/0 |
| 1.7 | Diosgenin* | 0/5 | 0/5 | 2/3 | 3/2 |
| 1.8 | L Valine | 0/5 | 0/5 | 1/4 | 5/0 |
| 1.9 | L Leucine | 0/5 | 5/0 | | |
| 1.10 | L Alanine | 0/5 | 0/5 | 2/3 | 5/0 |
| 1.11 | L Proline | 0/5 | 0/5 | 4/1 | 5/0 |
| 1.12 | Poly L Leucine mw 3-15,000 | 0/5 | 0/5 | 0/5 | 5/0 |
| 1.13 | Poly Valine mw 5-10,000 | 0/5 | 0/5 | 0/5 | 5/0 |
| 1.14 | Phloroglucinol* | 0/5 | 0/5 | 5/0 | |
| 1.Control | Untreated Controls | 0/60 | 0/60 | 1/60 | 17/43 |

It can be seen that the above organic compounds are effective at inducing ice formation during cooling of distilled water and that the ice nucleation occurs at significantly higher temperatures than those observed for distilled water. It can be seen that crystalline cholesterol is compound of choice.

Example Series II - Dose Response Studies

This example series is to demonstrate the minimum amount of cholesterol or leucine that is effective for nucleation within ampoules. All the experimental details are as for the first example series, except that the ampoules were coated with different amounts of either cholesterol or leucine. The results are shown in Table II. Examples 2.1 to 2.4 are in accordance with the invention; the others are comparative examples.

TABLE II

| Example | TREATMENT | Bath Temperature (°C) | | | |
|---|---|---|---|---|---|
| | | -2.5 | -5 | -7.5 | -10 |
| 2.1 | Cholesterol 0.5mg | 0/5 | 5/0 | | |
| 2.2 | Cholesterol 0.2mg/0 | 0/5 | 2/3 | 5/0 | |
| 2.3 | Cholesterol 0.1mg | 0/5 | 0/5 | 4/1 | 5/0 |
| 2.4 | Cholesterol 0.05mg | 0/5 | 0/5 | 0/5 | 2/3 |
| 2.5 | Leucine 0.5mg | 0/5 | 5/0 | | |
| 2.6 | Leucine 0.2mg | 0/5 | 2/3 | 4/1 | 4/1 |
| 2.7 | Leucine 0.1mg | 0/5 | 3/2 | 5/0 | |
| 2.8 | Leucine 0.05mg | 0/5 | 2/3 | 3/2 | 5/0 |
| 2.Control | Untreated Control | 0/20 | 0/20 | 2/18 | 6/14 |

It can be seen that 0.5 mg of leucine or cholesterol was effective to nucleate the freezing of distilled water in the ampoules at only 5°C below the freezing point.

Example Series III - Effectiveness of Cholesterol and Leucine in Ice Nucleation in Solutions Commonly Used in Cryopreservation

In this example series, the ability of organic ice nucleating agents to initiate ice crystal formation in various solution used in mammalian cell cryopreservation was studied. Only those employing crystalline cholesterol are in accordance with the invention. The solution aliquots (0.5 ml) were added to ampoules coated with either cholesterol (0.5 mg) or leucine (0.5 mg). Following a five minute equilibration period at 0°C, the ampoules were cooled as described in Experimental Series I. The results are shown in Table III.

## TABLE III

3.1)  Tissue  culture  medium  (Hanks  199  x1,  Flow laboratories)

| TREATMENT | Bath Temperature (°C) | | | |
|---|---|---|---|---|
| | -2.5 | -5 | -7.5 | -10 |
| Untreated ampoules | 0/5 | 0/5 | 0/5 | 1/4 |
| + Cholesterol | 0/5 | 4/1 | 5/0 | |
| + Leucine | 0/5 | 4/1 | 5/0 | |

3.2)  Tissue  culture  medium + foetal  calf  serum  (10% v/v)

| TREATMENT | Bath Temperature (°C) | | | | |
|---|---|---|---|---|---|
| | -2.5 | -5 | -7.5 | -10 | -12.5 |
| Untreated ampoules | 0/5 | 0/5 | 0/5 | 0/5 | 0/5 |
| + Cholesterol | 0/5 | 5/0 | | | |
| + Leucine | 0/5 | 1/4 | 3/2 | 4/1 | 5/0 |

3.3)  Tissue  culture  medium + serum  (10%  v/v)  + dimethylsulphoxide (10% v/v)

6

| TREATMENT | Bath Temperature (°C) | | | | | |
|---|---|---|---|---|---|---|
| | -2.5 | -5 | -7.5 | -10 | -12.5 | -15 |
| Untreated ampoules | 0/5 | 0/5 | 0/5 | 0/5 | 0/5 | 0/5 |
| + Cholesterol | 0/5 | 0/5 | 5/0 | | | |
| + Leucine | 0/5 | 0/5 | 0/5 | 2/3 | 4/1 | 5/0 |

3.4)   Tissue culture medium + dimethylsulphoxide (10% v/v) NB without serum

| TREATMENT | Bath Temperature (°C) | | | | |
|---|---|---|---|---|---|
| | -2.5 | -5 | -7.5 | -10 | -12.5 |
| Untreated ampoules | 0/5 | 0/5 | 0/5 | 0/5 | 0/5 |
| + Cholesterol | 0/5 | 0/5 | 5/0 | | |
| + Leucine | 0/5 | 0/5 | 0/5 | 3/2 | 5/0 |

3.5)   Tissue culture medium + serum (10% v/v) + glycerol (10% v/v)

| TREATMENT | Bath Temperature (°C) | | | |
|---|---|---|---|---|
| | -2.5 | -5 | -7.5 | -10 |
| Untreated ampoules | 0/5 | 0/5 | 0/5 | 0/5 |
| + Cholesterol | 0/5 | 0/5 | 5/0 | |
| + Leucine | 0/5 | 0/5 | 0/5 | 5/0 |

3.6) Tissue culture medium + serum (10% v/v) + methanol (10% v/v)

| TREATMENT | Bath Temperature ($^{\circ}$C) | | | | | |
|---|---|---|---|---|---|---|
| | -2.5 | -5 | -7.5 | -10 | -12.5 | -15 |
| Untreated ampoules | 0/5 | 0/5 | 0/5 | 0/5 | 0/50 | 0/5 |
| + Cholesterol | 0/5 | 0/5 | 0/5 | 5/0 | | |
| + Leucine | 0/5 | 0/5 | 0/5 | 0.5 | 4/1 | 5/0 |

3.7) Tissue culture medium + serum (10% v/v) + propanediol (10% v/v)

| TREATMENT | Bath Temperature ($^{\circ}$C) | | | | | |
|---|---|---|---|---|---|---|
| | -2.5 | -5 | -7.5 | -10 | -12.5 | -15 |
| Untreated ampoules | 0/5 | 0/5 | 0/5 | 0/5 | 0/5 | 1/4 |
| + Cholesterol | 0/5 | 0/5 | 3/2 | 5/0 | | |
| + Leucine | 0/5 | 0/5 | 2/3 | 4/1 | 5/0 | |

3.8) Tissue culture medium + serum (10% v/v) + polyvinylpyrrollidone (10% w/v)

| TREATMENT | Bath Temperature ($^{\circ}$C) | | | |
|---|---|---|---|---|
| | -2.5 | -5 | -7.5 | -10 |
| Untreated ampoules | 0/5 | 0/5 | 0/5 | 0/5 |
| + Cholesterol | 0/5 | 5/0 | | |
| + Leucine | 0/5 | 0/5 | 4/1 | 5/0 |

It can be seen that the presence of foetal calf serum seems to have a masking effect on the efficacy of leucine, and for that reason cholesterol is the solid of choice. The cryoprotectants are those that are routinely used in the art at present, and cholesterol was found to be effective in all cases.

Example Series IV - Effectiveness in Other Cryopreservation Containers

In addition to being effective in polypropylene tubes (Eppendorf) both crystalline cholesterol and leucine for comparison will nucleate ice in distilled water following coating onto the inside of the following other containers commonly used in cryopreservation:
CRYOTUBES (NUNC, Copenhagen, Denmark)
French Straws (IMV, L'Aigle, France) and
Glass Ampoules
Once coated, such containers may be sterilized by either autoclaving or heat drying within an oven. They still retain their ability to nucleate distilled water and other aqueous solutions during cooling. Data are not significantly different from those obtained with cholesterol in polypropylene tubes (Example Series I to III).

Example Series V - Effectiveness by the Addition of Coated Particles

Both crystalline cholesterol and leucine for comparison may be coated onto glass beads or microcarrier beads used for cell culture e.g. CYTODEX (Pharmacia). When such coated particles are added to biological solutions, they are effective in nucleating ice during cooling.

Example Series VI - Effectiveness for Yeast with and without Cryoprotectant

With the yeast Saccharomyces cerevisiae, ampoules treated with cholesterol (0.5 mg per ampoule) were used. 0.5 ml of a 24 hr cell suspension was added per ampoule and cooled rapidly to liquid nitrogen temperature, with no seeding of ice. On thawing the cell suspensions were diluted and viability was assayed by the ability to form colonies on agar. 5 ampoules were used per experimental treatment and viability determined from the average of 5 replicates per ampoule. The recovery (%) was:
a) No added cryoprotectant

| | |
|---|---|
| Untreated ampoules | 5.2 +/- 0.7 % |
| Treated ampoules | 10.4 +/- 1.2 % |

b) In the presence of Glycerol (1% v/v)

| | |
|---|---|
| Untreated ampoules | 35.5 +/- 4.4 % |
| Treated ampoules | 55.7 +/- 3.7 % |

Example Series VII - Effectiveness with Human Embryos

Multipronucleate human embryos were used and frozen to liquid nitrogen temperature in the presence of the cryoprotective additive, propanediol, using 1 embryo per tube, with no seeding of ice. On thawing viability was assessed by the ability to divide into 4 cells in tissue culture. When untreated ampoules were used 2 out of 6 could be recovered. When treated ampoules (ie containing 0.5 mg cholesterol per ampoule) were used 10 out of 12 could be recovered. The ampoules used were the same as for Example Series VI.

**Claims**

1. A method of cryopreserving a biological sample, comprising cooling the sample in an aqueous medium in contact with crystalline cholesterol which causes water in the aqueous medium to be nucleated at the freezing point of the medium or sufficiently close to the freezing point not to cause significant damage to the sample.

2. A method as claimed in claim 1 in which the cholesterol is sufficiently non-toxic to the biological sample.

3. A method as claimed in any one of claims 1 or 2 in which the cholesterol is at least partially coated on an inside surface of a suitable cryopreservation vessel.

4. A method as claimed in claim 3 in which the vessel is an ampoule, a cryopreservation tube or a cryopreservation straw.

5. A substrate at least partially on which crystalline cholesterol has been deposited by evaporation of solvent from a solution of cholesterol, the crystalline cholesterol capable of causing water in an aqueous medium to be nucleated at the freezing point of the medium or sufficiently close to the freezing point not to cause significant damage to a sample placed in the medium.

6. An aqueous cryopreservation medium for a biological sample, the medium comprising crystalline cholesterol which causes water in the aqueous medium to be nucleated at the freezing point of the medium or sufficiently close to the freezing point not to cause significant damage to the sample.

7. The use of crystalline cholesterol as an ice nucleator.

**Patentansprüche**

1. Verfahren zur Kältekonservierung einer biologischen Probe, welches das Abkühlen der Probe in einem wässrigen Medium in Kontakt mit kristallinem Cholesterin umfaßt, welches das Wasser veranlaßt, in einem wässrigen Medium am Gefrierpunkt des Mediums oder ausreichend nahe dem Gefrierpunkt mit Keimen versehen zu werden und keinen signifikanten Schaden der Probe zuzuführen.

2. Verfahren nach Anspruch 1, bei welchem das Cholesterin für die biologische Probe ausreichend nicht toxisch ist.

3. Verfahren nach einem der Ansprüche 1 oder 2, bei welchem das Cholesterin wenigstens teilweise an der Innenoberfläche eines passenden Kältekonservierungskessels als Schicht aufgebracht wird.

4. Verfahren nach Anspruch 3, bei welchem der Kessel eine Ampulle, ein Kältekonservierungsrohr oder ein Kältekonservierungshalm ist.

5. Ein Substrat, auf welchem wenigstens teilweise kristallines Cholesterin durch Evaporation eines Lösungsmittels einer Cholesterinlösung abgelagert ist, wobei das kristalline Cholesterin in der Lage ist, Wasser in einem wässrigen Medium beim Gefrierpunkt des Mediums oder entsprechend nahe dem Gefrierpunkt mit Keimen zu versehen und keinen signifikanten Schaden der in das Medium eingebrachten Probe zu verursachen.

6. Ein wässriges Kältekonservierungsmittel für eine biologische Probe, wobei das Mittel kristallines Cholesterin enthält, welches Wasser veranlaßt, in wässriger Lösung beim Gefrierpunkt des Mediums oder ausreichend nahe am Gefrierpunkt mit Keimen versehen zu werden und keinen signifikanten Schaden der Probe zu verursachen.

7. Verwendung von kristallinem Cholesterin als Eiskeimbildner.

**Revendications**

1. Procédé de cryoconservation d'un échantillon biologique, comprenant le refroidissement de l'échantillon dans un milieu aqueux en contact avec du cholestérol cristallin, qui provoque la nucléation de l'eau du milieu aqueux au point de congélation du milieu ou à un point suffisamment proche du point de congélation pour ne pas provoquer des dommages significatifs de l'échantillon.

2. Procédé suivant la revendication 1, dans lequel le cholestérol est suffisamment non toxique pour l'échantillon biologique.

3. Procédé suivant l'une quelconque des revendications 1 ou 2, dans lequel le cholestérol est au moins partiellement enduit sur une surface interne d'un récipient de cryoconservation approprié.

4. Procédé suivant la revendication 3, dans lequel le récipient est une ampoule, un tube de cryoconservation ou une paillette de cryoconservation.

5. Substrat sur lequel du cholestérol cristallin a, au moins partiellement, été déposé, par évaporation du solvant d'une solution de cholestérol, le cholestérol cristallin étant capable de provoquer la nucléation de l'eau d'un milieu aqueux au point de congélation du milieu ou suffisamment proche du point de congélation pour ne pas provoquer de dommages significatifs à un échantillon placé dans le milieu.

6. Milieu de cryoconservation aqueux pour un échantillon biologique, le milieu comprenant du cholestérol cristallin qui provoque la nucléation de l'eau du milieu aqueux au point de congélation du milieu ou à un point suffisamment proche du point de congélation du milieu pour ne pas provoquer de dommages significatifs de l'échantillon.

7. Utilisation du cholestérol cristallin comme agent de nucléation de la glace.